# FASCICULE DE BREVET EUROPEEN

(11) **EP 1 337 234 B1**
(45) Date de publication et mention de la délivrance du brevet: **19.01.2011**
(21) Numéro de dépôt: 01996360.2
(22) Date de dépôt: 12.11.2001
(51) Int. Cl.: A61K 31/19, A61K 31/4015, A61K 45/00, A61K 8/00, A61K 8/04, A61K 8/06, A61K 8/36, A61K 8/365, A61K 8/40, A61K 8/44, A61K 8/49, A61K 31/185, A61K 31/38, A61P 17/00, A61P 17/06, A61P 17/12, A61P 17/16, A61Q 19/00, A61Q 19/08, C07D 277/00

(54) **UTILISATION DE DERIVES DE L'ACIDE 2-OXOTHIAZOLIDINE 4-CARBOXYLIQUE COMME AGENTS PRODESQUAMANTS**
VERWENDUNG VON DERIVATEN DER 2-OXOTHIAZOLIDIN-4-CORBONSÄURE ALS PRÄ-ABSCHUPPUNGSMITTEL
USE OF 2-OXOTHIAZOLIDINE 4-CARBOXYLIC ACID DERIVATIVES AS AGENTS PROMOTING SKIN PEELING

(30) Priorité: 17.11.2000 FR 0014865
(43) Date de publication de la demande: 27.08.2003
(73) Titulaire: L'Oréal, 75008 Paris (FR)
(72) Inventeur: GALEY, Jean-Baptiste, F-93600 Aulnay-sous-Bois (FR); BERNARD, Dominique, F-75015 Paris (FR); SIMONETTI, Lucie, F-94300 Vincennes (FR)
(74) Mandataire: Brohmi, Karim
(86) Numéro de dépôt international: PCT/FR2001/003523
(87) Numéro de publication internationale: WO 2002/039976

(56) Documents cités:
- EP-A- 0 583 863
- EP-A- 0 655 245
- EP-A- 1 038 515
- FR-A- 2 767 833
- FR-A- 2 773 323
- US-A- 5 968 487
- US-A- 6 004 543
- US-A- 6 007 827

## Description

L'invention se rapporte à l'utilisation, dans une composition ou pour la fabrication d'une composition, d'au moins un dérivé de l'acide 2-oxothiazolidine 4-carboxylique le dérivé ou la composition étant destinés à favoriser la desquamation de la peau et/ou stimuler le renouvellement épidermique et/ou lutter contre le vieillissement intrinsèque de la peau.

L'invention se rapporte également à un procédé de traitement non thérapeutique de la peau destiné à favoriser la desquamation et/ou stimuler le renouvellement épidermique et/ou lutter contre le vieillissement intrinsèque de la peau consistant à appliquer sur la peau une composition comprenant au moins un dérivé de l'acide 2-oxothiazolidine 4-carboxylique.

Le vieillissement cutané résulte de deux processus distincts et indépendants qui font intervenir des facteurs intrinsèques ou extrinsèques.

Le vieillissement intrinsèque ou chronobiologique correspond au vieillissement « normal » ou physiologique lié à l'age.

Le vieillissement extrinsèque correspond au vieillissement provoqué d'une manière générale par l'environnement et plus particulièrement au photovieillissement dû à l'exposition au soleil, à la lumière ou à tout autre rayonnement (EP-A2-0 815 840, Kligman, A. M. et al., Journal of Cutaneous Aging and Cosmetic Dermatology, Vol. 1, N°.1, pp. 5-12 (1988)).

La présente invention ne s'intéresse qu'au vieillissement cutané intrinsèque ou physiologique.

Le vieillissement cutané en général se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté ou par l'apparition de télangiectasies.

Les changements de la peau dus au vieillissement intrinsèque sont la conséquence d'une sénescence génétiquement programmée où interviennent des facteurs endogènes. Ce vieillissement intrinsèque se traduit notamment par un ralentissement du renouvellement des cellules de l'épiderme et l'apparition de fines rides ou ridules.

Au contraire, le vieillissement extrinsèque résulte, au niveau du derme, de la dégradation des fibres de collagène ayant notamment pour conséquence des altérations cliniques telles que des rides épaisses et la formation d'une peau molle et tannée.

La desquamation est un phénomène naturel lié au fait que l'épiderme, qui constitue la couche supérieure de la peau, est en constante régénération.

L'épiderme humain est constitué de plusieurs assises de cellules dans lesquelles on trouve principalement quatre types de cellules: les kératinocytes, très majoritaires, les mélanocytes, les cellules de Langerhans et les cellules de Merkel. La répartition de ces cellules en plusieurs couches superposées explique le caractère stratifié de l'épiderme.

L'épiderme est conventionnellement divisé en une couche basale de kératinocytes qui constitue la couche germinative de l'épiderme, une couche dite épineuse constituée de plusieurs couches de cellules polyédriques disposées sur les cellules germinatives, une couche dite granuleuse constituée de cellules aplaties contenant des inclusions cytoplasmiques distinctes, les grains de kératohyaline, et enfin une couche supérieure appelée couche cornée (ou stratum corneum), constituée de kératinocytes au stade terminal de leur différenciation appelés coméocytes. Les cornéocytes sont des cellules momifiées, anucléées qui dérivent des kératinocytes et s'éliminent par desquamation. Cette perte en surface est compensée par la migration de cellules de l'assise basale vers la surface de l'épiderme. Il s'agit du renouvellement perpétuel de l'épiderme. Une élimination forcée de la couche cornée accélère le renouvellement et permet de lutter contre le vieillissement cutané.

Les cornéocytes sont principalement composés d'une matrice fibreuse contenant des cytokératines, entourée d'une structure très résistante de 15 nm d'épaisseur, appelée enveloppe cornée ou comifiée. L'empilement de ces cornéocytes constitue la couche cornée qui est responsable de la fonction de barrière de l'épiderme. Au cours du processus normal de desquamation, les cornéocytes les plus superficiels se détachent de la surface de l'épiderme.

Des structures intercellulaires dérivant des desmosomes, appelées coméosomes ou cornéodesmosomes, ont été décrites dans la couche cornée. Des études récentes ont montré leur importance majeure dans la cohésion intercornéocytaire ainsi que dans le processus de desquamation.

La cornéodesmosine, par ailleurs caractérisée dans la demande EP-A-0 972 042 de la Demanderesse, est une protéine de la couche cornée de l'épiderme, impliquée dans la cohésion intercornéocytaire et constitutive des cornéodesmosomes.

Dans la couche cornée, une corrélation étroite existe entre la dissociation cellulaire et la protéolyse de certains composants cornéodesmosomaux comme la desmogléine I et la cornéodesmosine. Plusieurs protéases à sérine de type trypsine ou chymotrypsine semblent être impliquées dans la protéolyse des coméodesmosomes, comme en particulier des protéases de type Chymotrypsine-like ou Trypsine-like (Lundström A., Egelrud T., J. Invest. Dermatol. 1988, 91:340-343 et 1990, 84:216-220).

On connaît dans l'art antérieur divers agents destinés à lutter contre le vieillissement cutané, particulièrement en favorisant la desquamation, c'est-à-dire l'élimination des cellules « mortes » situées à la surface de la couche cornée de l'épiderme. Cette propriété "desquamante" est aussi appelée, souvent à tort, propriété kératolytique.

Ainsi, le brevet US 4,603,146 décrit l'emploi d'acide rétinoïque et de ses dérivés dans des compositions cosmétiques, en vue de lutter contre le vieillissement cutané.

Par ailleurs, de nombreux brevets et publications (voir par exemple la demande EP-A-413 528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les β-hydroxyacides et plus spécialement l'acide salicylique ainsi que ses dérivés pour leur propriétés desquamantes (voir les documents WO-A-93/10756 et US 4,767,750).

FR-A-2 767 833 porte sur un mélange de polypeptides, des compositions les contenant et un procédé de traitement cosmétique destiné à diminuer la cohésion intercornéocytaire et donc à favoriser la desquamation.

Il n'en demeure pas moins que le désir de conserver une apparence jeune conduit toujours à la recherche incessante de nouveaux composés et/ou de nouvelles compositions permettant de maintenir ou d'améliorer l'apparence de la peau.

Certains actifs cosmétiques sont capables de stimuler la dégradation des protéines du cornéodesmosome et donc la desquamation, sans doute, comme on l'a vu précédemment, en favorisant l'activité de protéases impliquées dans ce processus.

Dans cet ordre d'idée, il a été décrit dans la demande de brevet EP-A2-0 852 949 (Shiseido) que des dérivés d'acides alpha aminés de type glycine favorise la dégradation de la desmogléine (protéine du cornéodesmosome)..

En recherchant les relations structure moléculaire/activité par un test *in vitro* de dégradation cornéodesmosomale, la Demanderesse a maintenant découvert de manière surprenante et inattendue qu'un dérivé de l'acide 2-oxothiazolidine 4-carboxylique est capable de stimuler la dégradation de la cornéodesmosine.

Ce dérivé de l'acide 2-oxothiazolidine 4-carboxylique peut donc constituer un excellent actif pour favoriser la desquamation de la peau et/ou stimuler le renouvellement épidermique et donc lutter contre le vieillissement intrinsèque de la peau.

Le but de la présente invention est donc de disposer de nouvelles compositions comprenant un dérivé de l'acide L-2-oxothiazolidine 4-carboxylique (encore appelé "proclystéine"), pour favoriser la desquamation de la peau et/ou stimuler le renouvellement épidermique et donc lutter contre le vieillissement intrinsèque de la peau.

Certes, l'utilisation de la procystéine a fait déjà l'objet de nombreuses études et brevets, notamment dans le but de protéger l'organisme contre différents stress. Ainsi, des brevets de l'université Cornell couvrent l'utilisation de la procystéine à des fins thérapeutiques et comme inducteur de glutathion (US 4,335,210; US 4,434,158; US 4,438,124 et US 4,647,751).

Des brevets de la société Clintec (EP 501 641, EP 501 637, EP 535 390 et US 5,214,062) décrivent également l'utilisation de la procystéine pour le traitement thérapeutique de désordres parmi lesquels on peut citer les désordres hépatiques, cardiaques et viraux.

En outre, il est connu du document EP-A-656 201 (brevet de la société Free Radical Sciences) d'autres esters de l'acide L-2-oxothiazolidine 4-carboxylique utilisés comme précurseur de cystéine et en association avec des stimulateurs de glutathion pour prévenir la chute des cheveux et stimuler la pousse de nouveaux cheveux.

Par ailleurs, les propriétés dépigmentantes de la procystéine ont été décrites dans le document EP-A-780 120.

Le document EP-A-1 038 515 mentionne l'utilisation de la procystéine :
- pour dépigmenter ou blanchir la peau, les poils et/ou les cheveux,
- pour prévenir la chute des cheveux et/ou stimuler la repousse des cheveux,
- pour prévenir ou traiter le photo-vieillissement et/ou le stress environnemental, en particulier en raison des propriétés anti-radicalaires de l'acide L-oxothiazolidine-4-carboxylique,
- et/ou pour prévenir ou traiter les peaux grasses, par exemple dans le traitement de l'acné.

En revanche, les propriétés prodesquamantes de l'acide L-2-oxothiazolidine 4-carboxylique n'étaient pas connues jusqu'à maintenant.

Ainsi, à la connaissance de la demanderesse, il n'a jamais été décrit dans l'art antérieur que l'acide L-2-oxothiazolidine 4-carboxylique est capable de stimuler la dégradation de la cornéodesmosine et peut donc constituer un excellent actif pour favoriser la desquamation de la peau et/ou stimuler le renouvellement épidermique et donc lutter contre le vieillissement cutané intrinsèque.

En outre, de nombreuses pathologies cutanées se caractérisent par la production d'une couche cornée épaissie et par une desquamation anormale, c'est-à-dire par une hyperkératose. Celle-ci peut survenir sur tout territoire anatomique cutané et dans des contextes cliniques très variés. Son substratum physiopathologique et sa cause sont variés.

A titre d'exemples on peut citer :
- la xérose (ou sécheresse cutanée),
- les ichthyoses,
- le psoriasis,
- certaines lésions tumorales bénignes ou malignes,
- les hyperkératoses réactionnelles.

Ainsi, l'acide L-2-oxothiazolidine 4-carboxylique est capable de stimuler la dégradation de la cornéodesmosine et peut donc constituer un excellent actif pour favoriser la desquamation de la peau et/ou stimuler le renouvellement épidermique et donc traiter les pathologies cutanées qui se caractérisent par la production d'une couche cornée épaissie et par une desquamation anormale.

L'invention a donc pour premier objet, l'utilisation de l'acide 2-oxothiazolidine 4-carboxylique répondant à la formule (I) suivante: 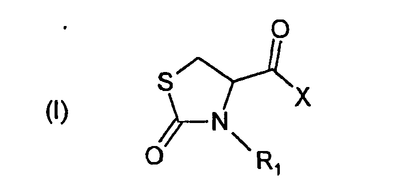 dans laquelle,
X représente un radical -OH, et R₁ représente
un atome d'hydrogène,
dans une composition cosmétique comprenant un milieu physiologiquement acceptable, en tant qu'agent destiné à favoriser la desquamation de la peau et/ou stimuler le renouvellement épidermique et donc avantageusement lutter contre le vieillissement intrinsèque de la peau.

L'invention concerne également l'usage des isomères optiques et/ou géométriques du dérivé de formule (I), seuls ou en mélange en toutes proportions, ainsi que les sels physiologiquement acceptables de ce dérivé.

Par milieu physiologiquement acceptable, on comprend un milieu compatible avec la peau, les muqueuses, les ongles le cuir chevelu et les cheveux.

Bien entendu, selon l'invention, les dérivés de l'acide 2-oxothiazolidine 4-carboxylique de formule (I) peuvent être utilisés seuls ou en mélange et en toute proportion.

Par la suite dans le texte, le terme dérivé de l'acide 2-oxothiazolidine 4-carboxylique de formule (I) s'entend comme désignant les dérivés ci-dessus décrits, d'origine naturelle ou synthétique, totalement ou partiellement purifiés ou toute préparation les contenant.

Par origine naturelle, on entend un dérivé extrait de matériel naturel dans lequel il se trouve présent. Par origine synthétique on entend un dérivé préparé par synthèse chimique ou par biotechnologie.

L'expression " totalement ou partiellement purifiés " signifie ici que, durant sa synthèse ou par rapport à son état naturel (plante ou cellules fraîches ou desséchées), le dérivé de l'acide 2-oxothiazolidine 4-carboxylique de formule (I), dans la composition de l'invention, a été concentré et/ou a été débarrassé, respectivement d'au moins une partie des produits réactionnels secondaires issus de sa synthèse ou d'au moins une partie des autres constituants de la plante. L'objet de l'invention concerne l'utilisation d'au moins un dérivé de l'acide 2-oxothiazolidine 4-carboxylique de formule (I) tel que défini précédemment, pour la fabrication d'une composition pharmaceutique ou dermatologique comprenant un milieu physiologiquement acceptable, la dite composition étant destinée à favoriser la desquamation de la peau et/ou stimuler le renouvellement épidermique et donc avantageusement lutter contre le vieillissement intrinsèque de la peau.

Un autre objet de l'invention concerne l'utilisation d'au moins un dérivé de l'acide 2-oxothiazolidine 4-carboxylique de formule (I) tel que défini précédemment, pour la fabrication d'une composition pharmaceutique ou dermatologique comprenant un milieu physiologiquement acceptable, la dite composition étant destinée à traiter les pathologies cutanées qui se caractérisent par la production d'une couche cornée épaissie et par une desquamation anormale, particulièrement la xérose ou sécheresse cutanée, les ichthyoses, le psoriasis, les lésions tumorales bénignes ou malignes, les hyperkératoses réactionnelles.

Parmi les dérivés de formule (I) utilisés selon l'invention, on préfère tout particulièrement
la procystéine ou acide L-2-oxo thiazolidine 4-carboxylique qui répond à la formule suivante:

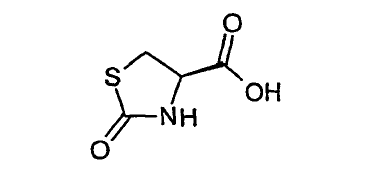

La quantité de dérivé de l'acide 2-oxothiazolidine 4-carboxylique de formule (I) utilisable selon l'invention, dépend bien évidemment de l'effet recherché et doit être en une quantité efficace pour favoriser la desquamation de la peau et/ou stimuler le renouvellement épidermique et donc lutter contre le vieillissement cutané intrinsèque.

A titre d'exemple la quantité de dérivé de l'acide 2-oxothiazolidine 4-carboxylique de formule (I) utilisable selon l'invention peut aller par exemple de 0,01% à 50% et de préférence de 0,1% à 10% du poids total de la composition.

Compte tenu de sa bonne solubilité dans l'eau (supérieure à 4%) et dans l'éthanol (supérieure à 10%), la procystéine procure l'avantage supplémentaire d'être facilement formulable à 1% dans un grand nombre de formulations cosmétiques de type émulsions E/H, H/E, liposomes, oléosomes à condition de maintenir le pH entre 5 et 8.

La composition peut être destinée à une application cosmétique ou pharmaceutique, particulièrement dermatologique.

La composition peut être ingérée, injectée ou appliquée sur la peau (sur toute zone cutanée du corps), les cheveux, les ongles ou les muqueuses (buccale, jugale, gingivale, génitale, conjonctive).

Selon le mode d'administration, la composition peut se présenter sous toutes les formes galéniques normalement utilisées, particulièrement en cosmétologie.

Une composition préférée est une composition cosmétique destinée à une application topique.

Pour une application topique sur la peau, la composition utilisable selon l'invention peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

La composition utilisable selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Les quantités des différents constituants des compositions utilisables selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits après-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Les compositions utilisables selon l'invention peuvent également consister en des préparations solides constituant des savons ou des pains de nettoyage.

Les compositions utilisables selon l'invention peuvent aussi être conditionnées sous forme d'une composition pour aérosol comprenant également un agent propulseur sous pression.

Lorsque la composition utilisable selon l'invention est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Lorsque la composition utilisable selon l'invention est une solution ou un gel huileux, la phase grasse peut représenter plus de 90% du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides têts que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Les compositions utilisables selon l'invention peuvent contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Les compositions utilisables selon l'invention peuvent associer au moins un dérivé de l'acide 2-oxothiazolidine 4-carboxylique de formule (I) à d'autres agents actifs. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique ;
- les agents anesthésiques tels que le chlorhydrate de lidocaïne et ses dérivés ;
- les agents antiprurigineux comme la thénaldine, la triméprazine ou la cyproheptadine ;
- les-agents agissant sur l'éclat du teint en favorisant le turn-over et la desquamation (agents kératolytiques) tels que les acides α- et β-hydroxycarboxyliques ou β-cétocarboxyliques, leurs sels, amides ou esters et plus particulièrement les hydroxyacides tels que l'acide glycolique, l'acide

lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un radical alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides.

Ainsi, selon un mode particulier, la composition utilisée selon l'invention comprend également au moins un agent choisi parmi les agents antibactériens, les antiparasitaires; les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents modulant la différenciation et/ou la prolifération et/ou la pigmentation cutanée, les extraits d'origine végétale, marine ou bactérienne ou leurs mélanges.

On peut également envisager que la composition utilisée selon l'invention comprenant au moins un dérivé de formule (I) tel que défini précédemment soit sous forme liposomée, telle que notamment décrite dans la demande de brevet WO 94/22468 déposée le 13 octobre 1994 par la société Anti Cancer Inc.

Selon un autre aspect, la composition comprend au moins l'association d'au moins un dérivé de l'acide 2-oxothiazolidine 4-carboxylique répondant à la formule (I) et d'au moins un autre agent prodesquamant.

Les autres agents prodesquamants sont des agents prodesquamants reconnus pour leurs propriétés hydratantes et/ou agissant sur l'éclat du teint en favorisant le turn-over et la desquamation (agents kératolytiques).

Les autres agents prodesquamants reconnus pour leurs propriétés hydratantes sont choisis parmi le glycérol et l'urée et leurs dérivés, l'acide pyrrolidone carboxylique, les sels d'ammonium de l'acide lactique.

Les autres agents prodesquamants agissant sur l'éclat du teint en favorisant le turn-over et la desquamation (agents kératolytiques) sont choisis parmi les hydroxyacides, en particulier les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique.

Selon encore un autre aspect, l'invention a pour objet un procédé de traitement non thérapeutique de la peau destiné à favoriser la desquamation de la peau et/ou stimuler le renouvellement épidermique, **caractérisé en ce qu**'on applique sur la peau une composition cosmétique comprenant au moins dérivé de l'acide 2-oxothiazolidine 4-carboxylique de formule (I) tel que défini précédemment.

L'invention a encore pour objet un procédé de traitement non thérapeutique pour lutter contre le vieillissement cutané intrinsèque, **caractérisé en ce qu**'on applique sur la peau une composition cosmétique comprenant au moins un dérivé de l'acide 2-oxothiazolidine 4-carboxylique de formule (I) tel que défini précédemment.

L'invention a encore pour objet un procédé pour favoriser la desquamation de la peau et/ou stimuler le renouvellement épidermique et donc lutter contre le vieillissement cutanée chez une personne ayant une desquamation de la peau anormalement bas et/ou un renouvellement épidermique anormalement bas, comprenant l'application topique sur la peau d'une quantité efficace d'au moins un dérivé de l'acide 2-oxothiazolidine 4-carboxylique de formule (I) tel que défini précédemment.

L'invention a encore pour objet un procédé pour favoriser la desquamation de la peau et/ou stimuler le renouvellement épidermique chez une personne ayant une production de couche cornée épaissie et une desquamation anormale comprenant l'application topique sur la peau d'une quantité efficace d'au moins un dérivé de l'acide 2-oxothiazolidine 4-carboxylique de formule (I) tel que défini précédemment.

L'invention est illustrée plus en détail dans les exemples suivants. Ces exemples ne sauraient limiter en aucune façon la portée de l'invention.

**Exemple 1 :** Méthode d'évaluation de la desquamation par la mesure de la dégradation des cornéodesmosines.

On étudie, dans cet exemple, la capacité de dérivés de l'acide 2-oxothiazolidine 4-carboxylique de formule (I) selon l'invention à favoriser la desquamation par la dégradation des cornéodesmosines.

La cornéodesmosine est un des marqueurs majoritaires de la desquamation au niveau du cornéodesmosome. Elle est étudiée sur immunoblot après séparation par électrophorèse et transfert sur membrane. Après un marquage spécifique avec l'anticorps monoclonal G36-19, elle est révélée par chimiluminescence. L'anticorps monoclonal murin G36-19 est spécifique de la cornéodesmosine, il fait partie d'une série d'anticorps dirigés contre des antigènes de différenciation épidermique, produits après immunisation d'une souris avec un homogénat de couche cornée plantaire humaine, puis caractérisé (Serre G. et al., J. Invest. Dermatol. 1991; 97(6):1061-72).

Des strippings vernis sont effectués sur le bas de jambes de volontaires (modification de la procédure de Lundström A. et Egelrud T. Acta Derm. Venereol. (stockh) 71, 471-474, 1991). Les feuillets nylon-vernis associés aux cornéocytes sont plongés dans de l'acétone 1ml/cm² afin de détacher les cornéocytes. Le mélange est filtré puis rincé trois fois avec le même volume d'acétone afin d'éliminer toute trace de vernis. Enfin le mélange est séché sous vide : on obtient alors des poudres acétoniques de *stratum corneum.*

Les poudres acétoniques sont aliquotées par 1 mg. 100 µl des solutions aqueuses contenant 2 % d'actif ajustées à un pH 8.0 sont ajoutés. Des témoins sans actif sont réalisés dans les mêmes conditions. Deux temps d'incubation sont étudiés : t=0 et t=17h. Dans ce dernier cas l'incubation a lieu à 30°C sous agitation.

Après l'incubation, les mélanges sont centrifugés 10 min à 10000g. Le surnageant est éliminé et remplacé par 100 µl de tampon Laemmli 0,0625 M Tris/HCl pH 6,8, 2% SDS, 200 mM DTT, 10% glycérol qui permet l'extraction des protéines. Le mélange est porté à ébullition 10 min à 100°C puis broyé au Potter. Le mélange est centrifugé 10 min à 10000g puis le surnageant est recueilli. Il contient les protéines du cornéodesmosome.

Les protéines totales sont dosées selon la méthode de Bradford (kit Biorad). Ceci permet un ajustement à 0.6 mg/ml des échantillons et une comparaison réelle des traitements.

Les échantillons ainsi qu'un étalon bas poids moléculaire Rainbow (Amersham Pharmacia Biotech) au 1/3 sont séparés par électrophorèse sur gel à 12% d'acrylamide pendant ½ h à 100V, puis 1h à 200V. Après l'électrophorèse, les protéines sont transférées sur membrane Immobilon-P (Millipore) pendant 3 h à 60V. La membrane est alors incubée 2 fois 15 min dans du tampon TBS-TL : Tris 25mM, 0,15M NaCl pH 7,2, 0,05%Tween 20, 0,5% Lait écrémé en poudre afin de bloquer les sites non spécifiques. L'incubation avec l'anticorps G36-19 au 1/12500 est réalisée overnight à 4°C. Après deux rinçages de 5 min dans le TBS-TL la membrane est incubée avec un anticorps goat anti-mouse IG(H+L) peroxydase conjugale (Biorad) au 1/4000 1h30 à température ambiante. Après plusieurs rinçages de 5 min dans du TBS-TL puis TBS (sans lait, ni Tween), la membrane est incubée 1 min dans 10 ml de réactif ECL (Amersham Pharmacia Biotech). La chemiluminescence des bandes de cornéodesmosine est mesurée avec le FluorS Multimager (Biorad). Les bandes de 33 et 46 kD sont quantifiées avec le logiciel Quantity-one (Biorad).

Les résultats de cette étude sont résumés dans le tableau suivant :

| **Effet de l'acide L-2-oxothiazolidine 4-carboxylique (procystéine) sur la dégradation des cornéodesmosines** | |
|---|---|
| Molécule testée | Pourcentage d'augmentation de la dégradation des cornéodesmosines |
| Contrôle | 0% |
| **procystéine** | 77% |

Le Contrôle correspond à un témoin réalisé avec le tampon de solubilisation sans actif dans les mêmes conditions du test. Ce contrôle prend en compte la dégradation naturelle des cornéodesmosines qui a lieu au cours de l'incubation.

Il apparaît clairement que la procystéine favorise la dégradation des cornéodesmosines.

### Exemple 2: Compositions

| Crème prodesquamante pour le visage | | |
|---|---|---|
| - Acide L-2-oxothiazolidine 4-carboxylique | | 2,00 % |
| - Stéarate de sodium | | 3,00 % |
| - Huile de vaseline | | 6,00 % |
| - Alkyl paraben | | 0,05 % |
| - Sorbate de potassium | | 10,00 % |
| - Alcool stéarylique | | 1,00 % |
| - Parfum | | 1,00 % |
| - Eau | qsp | 100,00 % |

| Crème prodesquamante pour le corps | | |
|---|---|---|
| - Acide L-2-oxothiazolidine 4-carboxylique | | 5,0 % |
| - Huile de jojoba | | 13,0 % |
| - Cire de sipol | | 6,0 % |
| - Palmitate d'isopropyle | | 2,0 % |
| - Glycérol | | 15,0 % |
| - Alkyl paraben | | 0,5 % |
| - Parfum | | 1,0 % |
| - Eau | qsp | 100,0 % |

| Crème de soin prodesquamante | | |
|---|---|---|
| - Acide L-2-oxothiazolidine 4-carboxylique | | 1 % |
| - Polyéthylène glycol 50 oxyéthyléné | | 3 % |
| - Mono diglycéryl stéarate | | 3 % |
| - Huile de vaseline | | 24 % |
| - Acool cétylique | | 5 % |
| - Eau | qsp | 100 % |

| Crème de soin desquamante pour le corps | | |
|---|---|---|
| - Acide L-2-oxothiazolidine 4-carboxylique | | 0,5 % |
| - Cire de sipol | | 6,0 % |
| - Glycéryl monostéarate | | 1,5 % |
| - Stéarate de sodium | | 0,8 % |
| **-** Huile de vaseline | | 6,0 % |
| - Palmitate d'isopropyle | | 2,0 % |
| - Glycérol | | 15,0 % |
| - Parfum | | 0,3 % |
| - Eau | qsp | 100,0 % |

| Crème de soin prodesquamante | | |
|---|---|---|
| - Acide L-2-oxothiazolidine 4-carboxylique | | 0,50 % |
| - Huile de jojoba | | 13,00 % |
| - Alkyl paraben | | 0,05 % |
| - Sorbate de potassium | | 0,30 % |
| - Cyclopenta diméthylsiloxane | | 10,00 % |
| - Alcool stéarylique | | 1,00 % |
| - Acide stéarique | | 4,00 % |
| - Stéarate de polyéthylène glycol | | 3,00 % |
| - Vitamine E | | 1,00 % |
| - Glycérol | | 3,00 % |
| - Eau | qsp | 100,00 % |

## Revendications

1. Utilisation d'au moins un dérivé de l'acide 2-oxothiazolidine 4-carboxylique répondant à la formule (I) suivante: 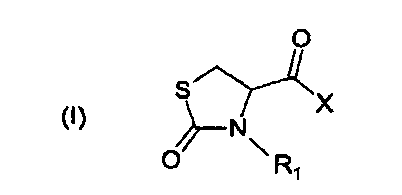 dans laquelle,
X représente un radical -OH et R₁ représente un atome d'hydrogène,
ainsi que ses sels physiologiquement acceptables et ses isomères optiques et/ou géométriques,
dans une composition cosmétique comprenant un milieu physiologiquement acceptable, en tant qu'agent destiné à favoriser la desquamation de la peau et/ou stimuler le renouvellement épidermique.

2. Utilisation selon la revendication précédente, **caractérisée en ce que** le dérivé ou la composition sont destinés à lutter contre le vieillissement intrinsèque de la peau.

3. Utilisation d'au moins un dérivé de l'acide 2-oxothiazolidine 4-carboxylique répondant à la formule (I) tel que défini dans la revendication 1, pour la fabrication d'une composition pharmaceutique ou dermatologique comprenant un milieu physiologiquement acceptable, la dite composition étant destinée à traiter les pathologies cutanées qui se caractérisent par la production d'une couche cornée épaissie et par une desquamation anormale.

4. Utilisation selon la revendication 3, **caractérisée en ce que** les pathologies cutanées sont choisies parmi la xérose ou sécheresse cutanée, les ichthyoses, le psoriasis, les lésions tumorales bénignes ou malignes, les hyperkératoses réactionnelles.

5. Utilisation selon l'une quelconque des revendications précédentes, **caractérisée en ce que** le dérivé de l'acide 2-oxothiazolidine 4-carboxylique de formule (I) est utilisé en une quantité allant de 0.01% à 50% du poids total de la composition.

6. Utilisation selon l'une quelconque des revendications précédentes, **caractérisée en ce que** le dérivé de l'acide 2-oxothiazolidine 4-carboxylique de formule (I) est utilisé en une quantité allant de 0,1% à 10% du poids total de la composition.

7. Utilisation selon l'une quelconque des revendications précédentes, **caractérisée en ce que** la composition comprend en outre au moins un agent actif choisi parmi les agents antibactériens, les antiparasitaires, les antifongiques, les antiviraux, les anti-inflammatoires, les antiprurigineux, les anesthésiques, les agents agissant sur l'éclat du teint en favorisant le turn-over et la desquamation, les anti-radicaux libres, les antiséborrhéiques, les antipelliculaires, les antiacnéiques et/ou les agents modulant la différenciation et/ou la prolifération et/ou la pigmentation cutanée, les extraits d'origine végétale, marine ou bactérienne ou leurs mélanges.

8. Procédé de traitement non thérapeutique pour lutter contre le vieillissement cutané intrinsèque, **caractérisé en ce qu'**on applique sur la peau une composition cosmétique comprenant au moins un dérivé de l'acide 2-oxothiazolidine 4-carboxylique de formule (I) tel que défini dans la revendication 1.

## Claims

1. Use of at least one 2-oxothiazolidine-4-carboxylic acid derivative corresponding to formula (I) below: 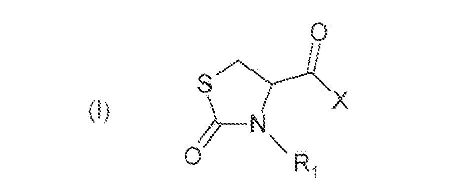 in which,
X represents an -OH radical and R₁ represents a hydrogen atom,
and also the physiologically acceptable salts thereof and the optical and/or geometrical isomers thereof,
in a cosmetic composition comprising a physiologically acceptable medium, as an agent for promoting desquamation of the skin and/or for stimulating epidermal renewal.

2. Use according to the preceding claim, **characterized in that** the derivative or the composition is intended to combat intrinsic aging of the skin.

3. Use of at least one 2-oxothiazolidine-4-carboxylic acid derivative corresponding to formula (I) as defined in Claim 1, for the manufacture of a pharmaceutical or dermatological composition comprising a physiologically acceptable medium, said composition being intended to treat skin pathologies **characterized by** the production or a thickened horny layer and by abnormal desquamation.

4. Use according to Claim 3, **characterized in that** the skin pathologies are chosen from xerosis or dryness of the skin, ichthyosis, psoriasis, benign or malignant tumoral lesions, and reactional hyperkeratosis.

5. Use according to any one of the preceding claims, **characterised in that** the 2-oxothiazolidine-4-carboxylic acid derivative of formula (I) is used in an amount ranging from 0.01% to 50% of the total weight of the composition.

6. Use according to any one of the preceding claims, **characterised in that** the 2-oxothiazolidine-4-carboxylic acid derivative of formula (I) is used in an amount ranging from 0.1% to 10% of the total weight of the composition.

7. Use according to any one of the preceding claims, **characterized in that** the composition also comprises at least one active agent chosen from antibacterial agents, antiparasitic agents, antifungal agents, antiviral agents, antiinflammatory agents, antipruriginous agents, anesthetics, agents acting on the radiance of the complexion by promoting turnover and desquamation, free-radical scavengers, antiseborrheic agents, antidandruff agents, antiacne agents and/or agents for modulating skin differentiation and/or proliferation and/or pigmentation, and extracts of plant, marine or bacterial origin, or mixtures thereof.

8. Nontherapeutic treatment process for combating intrinsic aging of the skin, **characterised in that** a cosmetic composition comprising at least one 2-oxothiazolidine-4-carboxylic acid derivative of formula (I) as defined in Claim 1 is applied to the skin.

## Patentansprüche

1. Verwendung mindestens eines Derivats der 2-Oxothiazolidin-4-carbonsäure, das der folgenden Formel (I) entspricht: 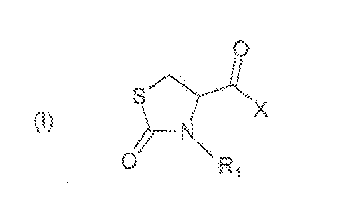 in der Formel:
X bedeutet eine Gruppe -OH und R₁ ist ein Wasserstoffatom,
sowie seiner physiologisch akzeptablen Salze und seiner optischen und/oder geometrischen isomere
in einer kosmetischen Zusammensetzung; die ein physiologisch akzeptables Medium enthält, als Stoff. der dazu vorgesehen ist,
die Abschuppung der Haut zu fördern und/oder die Erneuerung der Epidermis zu stimulieren.

2. Verwendung nach dem vorhergehenden Anspruch, **dadurch gekennzeichnet, dass** die Zusammensetzung dazu vorgesehen ist, die altersbedingte Hautalterung zu bekämpfen.

3. Verwendung mindestens eines Derivats der 2-Oxothiazolidin-4-carbonsäure, das der in Anspruch 1 definierten Formel (I) entspricht, für die Herstellung einer pharmazeutischen oder dermatologischen Zusammensetzung, die ein physiologisch akzeptables Medium enthält, wobei die Zusammensetzung dazu vorgesehen ist. Hauterkrankungen zu behandeln, die durch die Bildung einer verdickten Hornzellschicht und durch eine anormale Abschuppung gekennzeichnet sind.

4. Verwendung nach Anspruch 3, **dadurch gekennzeichnet, dass** die Hauterkrankungen unter Xerodermie oder Hauttrockenheit, Ichthyosis, Psoriasis, benignen oder malignen tumoralen Läsionen und reaktiven Hyperkeratosen ausgewählt sind.

5. Verwendung nach einem der vorhergehenden Ansprüche,
**dadurch gekennzeichnet, dass** das Derivat der 2-Oxothiazolidin-4-carbonsäure der Formel (I) in einem Mengenanteil von 0,01 bis 50 % des Gesamtgewichts der Zusammensetzung verwendet wird.

6. Verwendung nach einem der vorhergehenden Ansprüche,
**dadurch gekennzeichnet, dass** das Derivat der 2-Oxothiazolidin-4-carbonsäure der Formel (I) in einem Mengenanteil von 0,1 bis 10 % des Gesamtgewichts der Zusammensetzung verwendet wird.

7. Verwendung nach einem der vorhergehenden Ansprüche,
**dadurch gekennzeichnet, dass** die Zusammensetzung ferner mindestens einen wirkstoff enthält, der unter den antibakteriellen Wirkstoffen, antiparasitären Wirkstoffen, antimykotischen Wirkstoffen, antiviralen Wirkstoffen, entzündungshemmenden Wirkstoffen, Anästhetika, Wirkstoffen, die das Strahlen des Teints beeinflussen, indem der Turnover und die Abschuppung gefördert werden, Radikalfängern für freie Radikale, Antiseborrhoeika, Antischuppenmitteln und/oder Wirkstoffen, die die Differenzierung und/oder Proliferation und/oder die Pigmentierung der Haut modulieren, Extrakten pflanzlicher, mariner oder bakterieller Herkunft oder deren Gemischen ausgewählt ist.

8. Verfahren zur nichttherapeutischrn Behandlung zur Bekämpfung der altersbedingten Hautalterung, **dadurch gekennzeichnet, dass** eine kosmetische Zusammensetzung, die mindestens ein Derivat der 2-Oxothiazolidin-4-carbonsäure enthält, das der in Anspruch 1 definierten Formel (I) entspricht, auf die Haut aufgetragen wird.
